Europäisches Patentamt

European Patent Office

Office européen des brevets

(19)

(11) Publication number: **0 244 719 B1**

(12) **EUROPEAN PATENT SPECIFICATION**

(45) Date of publication of patent specification: **28.12.94**

(51) Int. Cl.⁵: **C07K 9/00**, //A61K37/02

(21) Application number: **87106051.3**

(22) Date of filing: **25.04.87**

The file contains technical information submitted after the application was filed and not included in this specification

(54) **Complex immunogen containing synthetic peptides.**

(30) Priority: **28.04.86 US 856909**

(43) Date of publication of application:
**11.11.87 Bulletin 87/46**

(45) Publication of the grant of the patent:
**28.12.94 Bulletin 94/52**

(84) Designated Contracting States:
**AT BE CH DE ES FR GB GR IT LI LU NL SE**

(56) References cited:
**EP-A- 0 109 942**
**EP-A- 0 154 902**
**WO-A-86/04065**

**VACCINES 87, 1987, pages 33-37, Cold Spring Harbor Laboratory; A.R. NEURATH etal.: "Multimeric immunogens containing several synthetic peptide analogs of thehepatitis-B virus envelope protein"**

**SCIENCE, vol. 224, 27 April 1984, pages 392-395; A.R. NEURATH et al.: "Locationand chemical synthesis of a pre-S gene coded immunodominant epitope ofhepatitis B virus"**

(73) Proprietor: **New York Blood Center, Inc.**
**310 East 67 Street**
**New York, New York 10021 (US)**

Proprietor: **CALIFORNIA INSTITUTE OF TECHNOLOGY**
**1201 East California Boulevard**
**Pasadena**
**California 91125 (US)**

(72) Inventor: **Neurath, Alexander Robert, Dr.,**
**230, East 79 Street,**
**New York**
**New York 10021, (US)**
Inventor: **Kent, Stephen B.H., Dr.,**
**615, West California Boulevard,**
**Pasadena**
**California 91105, (US)**
Inventor: **Strick, Nathan,**
**619, Grassmere Terrace,**
**New York**
**New York 11691, (US)**

(74) Representative: **Cohausz & Florack Patentanwälte**
**Postfach 33 02 29**
**D-40435 Düsseldorf (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

PROC. NATL. ACAD. SCI. USA, vol. 79, December 1982, pages 7871-7875; A.R.NEURATH et al.: "Specificity of antibodies elicited by a synthetic peptidehaving a sequence in common with a fragment of a virus protein, the hepatitis Bsurface antigen"

CELL, vol. 46, no. 3, 1st August 1986, pages 429-436; A.R. NEURATH et al.:"Indentification and chemical synthesis of a host cell receptor binding site onhepatitis B virus"

Immunology Today, 1987, Vol.8(11), pp333-338

## Description

The present invention concerns a complex immunogen containing one or more distinct synthetic peptides. More particularly, the present invention concerns a saponin derivative-containing core structure containing several synthetic peptide analogs of the hepatitis B virus envelope protein.

In order to have a potential as vaccines, synthetic peptide analogs of viral proteins, known to elicit protective antibodies, must contain not only binding sites for such antibodies, but also domains reacting with T-cell receptors and Ia antigens (J. Berzofsky, "Antigenic Structural Requirements for MHC-Restricted Helper T Cell Recognition", Modern Approaches to Vaccines, 21-23, Edited by R. Chanock, R. Lerner & F. Brown, Cold Spring Harbor, NY: Cold Spring Harbor Laboratory, (1985); J. Berzofsky, "Intrinsic and Extrinsic Factors in Protein Antigenic Structure", Science, 229, 932-940, (1985); T. Watts, J. Gariepy, G. Schoolnik, H. McConnell, "T-Cell Activation by Peptide Antigen: Effect of Peptide Sequence and Method of Antigen Presentation", Proceedings of the National Academy of Sciences, U.S.A., 82, 5480-5484, (1985)).

The usefulness of synthetic peptides as immunogens for active prophylaxis may potentially be limited by the narrower genetic restriction of immune responsiveness to peptides as compared with the response to proteins. (A. R. Neurath, S. B. H. Kent, N. Strick, D. Stark and P. Sproul, "Genetic Restriction of Immune Responsiveness to Synthetic Peptides Corresponding to Sequences in the Pre-S Region of the Hepatitis B Virus (HBV) Envelope Gene", Journal of Medical Virology, 17, 119-125, (1985)).

It is also of concern that protein carriers and adjuvants (i.e., complete and incomplete Freund's adjuvant) which are unsuitable for human vaccines were used in many experiments aiming at the establishment of the prophylactic potential of synthetic peptides.

It would be advantageous to overcome these problems. An aim of the present invention is to overcome these problems by preparing polymeric synthetic immunogens containing multiple copies of several distinct synthetic peptides. Such polymers are more likely to have sites reacting with T-cell receptors and Ia antigens. The immune response to a polymer containing several distinct peptide analogs is also expected to be less restricted genetically than the response to individual peptides. Furthermore, the multimeric immunogen should have built-in adjuvanticity and should not require protein carriers (A. R. Neurath, S. B. H. Kent and N. Strick, "Location and Chemical Synthesis of a Pre-S Gene Coded Immunodominant Epitope of Hepatitis B Virus, Science, 224, 392-395, (1984); A. R. Neurath, S. B. H. Kent and N. Strick, "Antibodies to Hepatitis B Surface Antigen (HBsAg) Elicited by Immunization with a Synthetic Peptide Covalently Linked to Liposomes", The Journal of General Virology, 65, 1009-1014, (1984); A. R. Neurath, S. B. H. Kent and N. Strick, "Immune Response to Hepatitis-B Virus Determinants Coded by the Pre-S Gene", Vaccines '85, 185-189, Edited by R. A. Lerner, R. M. Chanock & F. Brown, Cold Spring Harbor, NY Cold Spring Harbor Laboratory, (1985)).

Previous studies indicate that the immune response to synthetic peptides can be enhanced by incorporating the synthetic peptides into liposomes (A. R. Neurath, S. B. H. Kent and N. Strick, "Antibodies to Hepatitis B Surface Antigen (HBsAg) Elicited by Immunization with a Synthetic Peptide Covalently Linked to Liposomes", The Journal of General Virology, 65, 1009-1014, (1984); A. R. Neurath, S. B. H. Kent and N. Strick, "Immune Response to Hepatitis-B Virus Determinants Coded by the Pre-S Gene", Vaccines '85, 185-189, edited by R. A. Lerner, R.M. Chanock and F. Brown, Cold Spring Harbor, NY: Cold Spring Harbor Laboratory) or into micelles (T. P. Hopp, "Immunogenicity of a Synthetic HBsAg Peptide: Enhancement by Conjugation to a Fatty Acid Carrier", Molecular Immunology, 21 13-16 (1984) and that the need for protein carriers can be obviated. However, in both cases Freund's adjuvant was used to obtain good antibody responses.

Immunostimulating complexes (iscoms) in which virus membrane proteins are presented in a multimeric form on a matrix of a saponin derivative Quil A (Spikoside) have been described recently (R. Morein, B. Sundquist, S. Hoglund, K. Dalsgaard and A. Osterhaus, "Iscom, A Novel Structure for Antigenic Presentation of Membrance Proteins From Enveloped Viruses", Nature, 308, 457-460, (1984); A. Osterhaus, K. Weijer, F. Uytdehaag, O. Jarrett, B. Sundquist and B. Morein, "Induction of Protective Immune Response in Cats by Vaccination with Feline Leukemia Virus Iscom", The Journal of Immunology, 135, 591-596, (1985)). The virus proteins incorporated into iscoms became highly immunogenic in the absence of adjuvants. These two articles concern virus disrupted by a detergent, not synthetic peptides. The disrupted virus is then placed in a vessel containing saponin derivative Quil A and the vessel is subjected to centrifugation. Thereafter, dialysis is performed.

EP-A-0109942, published on May 30, 1984, in Example 19, purports to incorporate into an ISCOM a synthetic peptide coresponding to an immunogenic determinant. According to the general teachings of this reference, the first step in the preparation of such ISCOMs would be to couple the synthetic peptide to a linking moiety. Thereafter, the product of the first step is complexed with glycoside, e.g. saponin. The inventor on EP-A-0109942 (Morein), along with other investigators, later reported in an article published in Immunology Today, 8 (1), 1987, pages 333-338, that the same preparation method idsclosed in EP-A-0109942, produced synthetic peptide ISCOMs that were poorly immunogenic. The investigators characterize the preparation method as not being powerful enough to prepare ISCMs containing more than 4-6 synthetic peptide molecules per complex molecule without causing polymerization of the peptide or the carrier. The poor immunogenicity may also be partly due to the fact that the ISCOMs prepared by Morein et al. were relatively small molecules, being on the order of 35 nm in diameter.

SUMMARY OF THE INVENTION

The present invention concerns a process to produce a complex immunogen comprising multiple copies of one or more distinct synthetic peptides covalently linked to a core structure comprising a saponin derivative and an organic compound or hydrophobic peptide having 12 to 24 carbon atoms and having an active moiety, the active moiety being either -COOH, -CHO, -NH$_2$ or -SH, and the products produced by this process as well as the use of such products in the preparation of a vaccine protective against viral infections.

The synthetic peptides can be synthetic peptide analogs of the hepatitis B virus envelope protein. The synthetic peptide analogs of the hepatitis B virus envelope protein can be derived from the three following distinct regions of the hepatitis B virus (HBV) envelope protein:

(1) the pre-S1 region,
(2) the pre-S2 region, and
(3) the S region.

The process for preparing this complex immunogen is characterized in that:

(1) in a first step there is linked in a water-miscible organic solvent
    (a) a saponin derivative to
    (b) an organic compound selected from the group consisting of a $C_{7-24}$-organic compound and a hydrophobic peptide, said organic compound containing one or more active groups selected from the group consisting of -COOH, -CHO, -NH$_2$, and -SH, and said organic compound being soluble in the water-miscible organic solvent;

(2) in a second step the complex formed in (1) is separated by gel filtration from the organic solvent and any excess saponin derivative; and

(3) in a third step one or more synthetic peptides are covalently linked to the linking moiety in the complex separated in (2) through the active moieties, said synthetic peptides corresponding to one or more antigenic determinants.

BRIEF DESCRIPTION OF THE DRAWINGS

Fig. 1 is a photomicrograph of a complex according to the present invention.

Fig. 2a and Fig. 2b are graphs of antibody responses in rabbits immunized with a complex according to the present invention.

Fig. 3 is a graph which represents the time course of antibody response in rabbits immunized with a complex according to the present invention.

Fig. 4 shows amino acid sequence of the translational products of the pre-S gene region deduced from sequences of HBV DNA. The sequences are presented in one-letter amino acid code words (such code words are defined in the Definitions herein). Sequences for five distinct HBV subtypes are presented. The 6th bottom line shows amino acid residues common to all five subtypes.

DEFINITIONS

| Amino Acid Code Words (as appearing in Fig. 4) | | |
|---|---|---|
| D | Asp | aspartic acid |
| N | Asn | asparagine |
| T | Thr | threonine |
| S | Ser | serine |
| E | Glu | glutamic acid |
| Q | Gln | glutamine |
| P | Pro | proline |
| G | Gly | glycine |
| A | Ala | alanine |
| C | Cys | cysteine |
| V | Val | valine |
| M | Met | methionine |
| I | Ile | isoleucine |
| L | Leu | leucine |
| Y | Tyr | tyrosine |
| F | Phe | phenylalanine |
| W | Trp | tryptophane |
| K | Lys | lysine |
| H | His | histidine |
| R | Arg | arginine |
| Synthetic Peptide - a peptide which is not naturally occurring | | |

## DETAILED DESCRIPTION OF THE INVENTION

The present invention concerns a complex (core structure) of a saponin derivative and an organic compound, i.e., an aliphatic compound or an organic compound, or a hydrophobic peptide containing one or more of the following active groups: carboxyl, aldehyde, alkyl amine and/or thiol groups. Any aliphatic compound or aromatic compound or hydrophobic peptide can be used, so long as they contain one of the above described active groups and are soluble in water-miscible organic solvents such as dimethyl sulfoxide, dimethyl formamide, ethanol, etc. The complex is then subjected to gel filtration to remove the organic solvent and excess of the saponin derivative. Then one or more distinct synthetic peptides are covalently linked to the complex. The peptides are specifically covalently linked to the active groups of the aliphatic compound. A preferred saponin derivative for use in this invention is Quil A.

Saponin ($C_{32}H_{54}O_{18}$; molecular weight = 726.5) is a plant glucoside derived from soapwort, quillaia and especially from the heartwood of Mora excelsa (British Guiana). Each saponin consists of a sapogenin which constitutes the algucon moiety of the molecule and a sugar. The sapogenin may be a steroid or a triterpene and the sugar moiety may be glucose, galactose, a pentose, or a methylpentose.

The aliphatic compound has 7 to 24 carbon atoms, preferably 12 to 18 carbon atoms and most preferably 14 to 18 carbon atoms and has one or more active moieties selected from the group -COOH, -CHO, $-NH_2$ and -SH. Non-limiting examples of fatty acids (saturated and unsaturated) for use in the present invention include stearic acid, oleic acid, palmitic acid and myristic acid, which can be activated with a carbodiimide, e.g., N-ethyl-N'(dimethylaminopropyl) carboiimide. A preferred aliphatic compound is octadecanethiol.

The peptide binds to the active site on the aliphatic compound. For example, a $-NH_2$ group at one end of the peptide (N-terminal end) can bind with a -COOH or -CHO active site on the complex. Alternatively, the -COOH end of a peptide can bind with a $-NH_2$ active site on the complex. Still further, a C-terminal homoserine lactone end of a peptide can bind with an amine site on the complex (T. Kempe, S. B. H. Kent, F. Chow, S. M. Peterson, W. I. Sundquist and J. J. L'Italien, Gene, 39, 239, (1985)). A sulfhydryl group in the peptide (=cysteine) can bind to a sulfhydryl group in the complex (containing, for example, oc-tadecanethiol).

5

The present invention is particularly directed to a complex immunogen containing multiple copies of three distinct synthetic peptides, derived from the pre-S1, pre-S2 and S-regions, respectively, of the hepatitis B virus (HBV) envelope (env) protein. Applicants have found that stimulating complexes according to the present invention, without additional adjuvants, elicited in rabbits antibodies recognizing the native HBV env protein.

Complexes according to the present invention containing several distinct peptide analogs offer the opportunity to: (1) overcome potential problems inherent in single-peptide immunogens, i.e., insufficient T-B cell cooperation during the immune response and narrow genetic restriction of immune responsiveness; (2) design of vaccines containing key epitopes involved in virus neutralization and protection against infection and (3) study the regulation of the immune response to distinct epitopes in genetically defined animals and to evaluate the mutal influences of individual epitopes on site-specific antibody responses.

A particularly preferred peptide for use in the present invention is pre-S (120-145) (A. R. Neurath, S. B. H. Kent, K. Parker, A. M. Prince, N. Strick, B. Brotman and P. Sproul, "Antibodies to a Synthetic Peptide from the Pre-S(120-145) Region of the Hepatitis B Virus Envelope are Virus-Neutralizing", Vaccine, 4, 35-37, (1986)). Also preferred are peptides pre-S(21-47) and S(135-155) derived from the pre-S and S sequences, respectively, of the HBV env protein.

The cloning and sequencing of the genome of several hepatitis virus (HBV) isolates led to the elucidation of the genetic structure of the viral DNA (P. Tiollais, P. Charnay, G. N. Vyas, Science, 213, 406, (1981)).

The immunologic markers of hepatitis B virus (HBV) infection include the surface antigen (HBsAg), which includes the S protein and the pre-S protein, the core antigen (HBcAg), the "e" antigen (HBeAg) and their respective antibodies. Antibodies against the S protein and the pre-S protein of HBsAg are protective against HBV infection.

Several antigenic subtypes of HBV and of subviral approximately 22 nm diameter particles (hepatitis B surface antigen; HBsAg) have been recognized (G. Le Bouvier, A. Williams, Am. J. Med. Sci., 270, 165 (1975)). All of these subtypes (for example, ayw, adyw, adw2, adw and adr) share common (group-specific) envelope epitopes, the immune response against which appears sufficient for protection against infection by any of the virus subtypes (W. Szmuness, C. E. Stevens, E. J. Harley, E. A. Zang, H. J. Alter, P. E. Taylor, A. DeVera, G. T. S. Chen, A. Kellner, et al., N. Engl. J. Med., 307, 1481, (1982)).

The HBV genome is composed of two DNA strands, namely the long (L) strand and the short (S) strand. The L strand transcript has four open reading frame regions which are termed (S + pre-S), C, P and X.

The open reading frame region (S + pre-S) corresponds to the envelope (env) gene of HBV DNA and codes for a family of proteins found in the HBV envelope and in virus related particles.

A schematic representation of the potential translation products of the env gene(s) of HBV DNA is as follows:

```
              Pre-S Region              S Region              400

        ├─────────────────────────────┤ ├────────────────────────┤

        1     12          120      174 . 175                   400


        ├──────────────────────────────┤ ├────────────────────────┤
        pre-S(1)  pre-S(12)  pre-S(120)  pre-S(174)  S(1)        S(226)

                                              175                 400
```

S region only:

$\vdash$————————————$\dashv$ S(226)

400

$\vdash$————————————————————————$\dashv$ pre-S(120)   S(226)

400

$\vdash$————————————————————————————$\dashv$ pre-S(12)   S(226)

400

$\vdash$————————————————————————————————$\dashv$ pre-S(1)   S(226)

The numbers in the above schematic refers to amino acids (AA). A translation initiation site at Met 1 exists for the adw$_2$ and adr substypes only. The first amino acid for the other subtypes correspond to position pre-S 12.

Hereinafter, amino acid sequences corresponding to the pre-S region (env 1 to 174) are designated with the prefix "pre-S" and amino acid sequences corresponding to the S region (env 175 to 400) are designated by the prefix "S". In the env gene product representation, the S region spans amino acids 175 to 400, as compared to amino acids 1 to 226 in the "S region only" representation.

In the above schematic, the pre-S region is defined by amino acid sequence positions pre-S 1 to amino acid sequence position pre-S 174. The S region is defined by sequence positions S 1 (amino acid 175 of the open reading frame and adjacent to pre-S 174) to sequence position S 266 (amino acid 400 of the open reading frame). The S-gene product (S-protein) consists of this 226 amino acid sequence.

The pre-S1 region includes pre-S 1 to pre-S 119 and the pre-S2 region includes pre-S 120 to pre-S 174.

The naturally occurring envelope proteins of hepatitis B virus include the following:

(1) a full length translational product of the env gene of HBV, i.e., for adw$_2$ and adr pre-S(1-174) + S-(175-400) = 400 amino acids, for ayw, adyw and adw pre-S(12-174) + S(1-226) = 389 amino acids (env 12-400);

(2) pre-S(120-174) + S(175-400) = 281 amino acids (env 120-400) = terminal 55 amino acids in the pre-S region + 226 amino acids comprising the entire S region (the corresponding proteins approximately are 33 and 36 kD in size (P33 and P36), and differ from each other in the extent of glycosylation); and

(3) S(1-226) = 226 amino acids, i.e., the entire S region (env 175-400); representing the approximately 22 and 26 kD major constituents of the HBV envelope (P22 and P26) in their non-glycosylated and glycosylated forms (the "S-protein").

Besides previously mentioned pre-S(21-47), pre-S(120-145) and S(135-155), preferred peptides according to the present invention include the following:

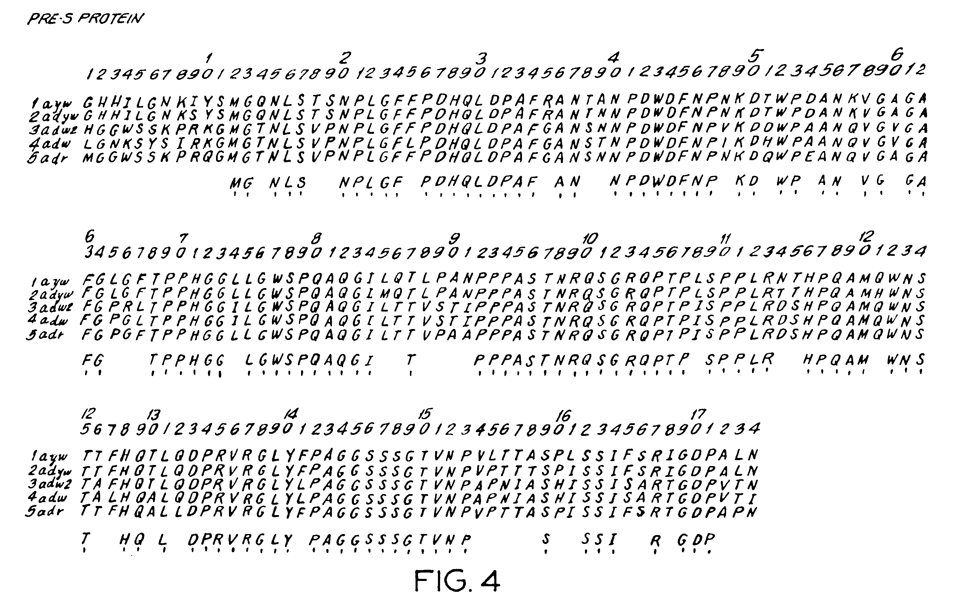

(1) pre-S(12-32), wherein the sequence is (see Fig. 4) MGTNLSVPNPLGFFPDHQLDP for subtype adw$_2$;

(2) pre-S(120-145), wherein the sequence is (see Fig. 4) MQWNSTAFHQTLQDPRVRGLYLPAGG for subtype adw$_2$;

(3) pre-S(32-53), wherein the sequence is (see Fig. 4) PAFGANSNNPDWDFNPVKDDWP for subtype adw$_2$;

(4) pre-S(117-134), wherein the sequence is (see Fig. 4) PQAMQWNSTAFHQTLQDP for subtype adw$_2$;

(5) pre-S(94-117), wherein the sequence is (see Fig. 4) PASTNRQSGRQPTPISPPLRDSHP for subtype adw$_2$;

(6) pre-S(153-171), wherein the sequence is (see Fig. 4) PAPNIASHISSISARTGDP for subtype adw$_2$;

(7) pre-S(1-21), wherein the sequence is (see Fig. 4) MGGWSSKPRKGMGTNLSVPNP for subtype adw$_2$;

(8) pre-S(57-73), wherein the sequence is (see Fig. 4) QVGVGAFGPRLTPPHGG for subtype adw$_2$;

(9) pre-S(1-11),

a. for adw$_2$, wherein the sequence is (see Fig. 4) MGGWSSKPRKG

b. for adr, wherein the sequence is (see Fig. 4) MGGWSSKPRQG.

Any analogs of the pre-S and S gene coded sequences of the present invention involving amino acid deletions, amino acid replacements, such as replacements by other amino acids, or by isosteres (modified amino acids that bear close structural and spatial similarity to protein amino acids), amino acid additions, or isosteres additions can be utilized, so long as the sequences elicit antibodies recognizing the pre-S protein of HBV or hepatitis B surface antigen.

In the formation of a peptide derived from natural sources, a protein containing the required amino acid sequence is subjected to selective proteolysis such as by splitting the protein with chemical reagents or using enzymes. Synthetic formation of the peptide requires chemically synthesizing the required chain of amino acids.

It is preferred to insure that the peptide residue corresponds to at least six consecutive amino acids within the pre-S or S gene coded region of hepatitis B virus and has the steric configuration to be recognized by antibody to hepatitis B virus. To this end, the given chain of amino acids may have bonded thereto as part of the amino acid chain, one or more additional amino acids on either, or both sides thereof. These additional amino acids can serve as auxiliary amino acids to enhance the stabilization of the amino acid chain so that it is readily recognized by antibody to hepatitis B virus. The additional amino acids can be the same amino acids in the same sequence as they occur in the natural protein, or other amino acids may be employed.

In one form of the invention, the peptide having a chain length of minimally six amino acids can be bounded on either side thereof with additional amino acids, e.g., three amino acids on either side of the residue, to form a longer chain of amino acids. The chain of amino acids may contain more than one amino acid sequence corresponding to at least six consecutive amino acids within the pre-S region of the surface antigen of hepatitis B virus.

The length of the individual amino acid sequence would depend on the method of producing the sequence. If the sequence is made by assembling individual amino acids by chemical means, then the sequence length would generally not exceed 50 amino acids, and preferably would not exceed 40 amino acids. If the synthetic peptide is obtained from a DNA route, the chain length could be longer, for example, 100 or more amino acids. It is, however, normally shorter, and optimally considerably shorter than the natural pre-S protein. Thus, in the embodiment wherein the peptide has units of both the S region and pre-S region, its peptide portions corresponding to the S region is shorter than the natural S protein, e.g., no more than 100 amino acids, preferably no more than 40 amino acids and usually less than 30 amino acids. In such cases, the peptide portion corresponding to the pre-S region can be of a length corresponding to the entire pre-S region, but generally is less than the entire pre-S region.

Where, however, the amino acid sequence is part of a long chain, such as when there are more than one sequence of amino acids, the chain can contain residues of various moieties, for example, segments of polyamino acids or polysaccharides.

In addition to containing one or more different or the same sequences of amino acids corresponding to at least six consecutive amino acids within the pre-S region of hepatitis B virus, e.g., containing more than one sequence of amino acids corresponding to different epitopes (antigenic determinants) in the pre-S region of hepatitis B virus, the immunogen of the present invention can contain amino acid chains containing epitopes of different antigens or allergens so as to be able to be used in a vaccine directed to hepatitis B virus and to one or more additional diseases, e.g., measles, influenza, smallpox, polio, diptheria, just to name a few. Such additional amino acid sequences can be of varying amino acid chain lengths.

There is realized by the present invention an immunogen which is characterized by the absence of naturally occuring envelope proteins of hepatitis B virus, i.e., the immunogens of the present invention are composed of one or more peptide sequences corresponding to a limited portion of the hepatitis B virus envelope protein. The immunogens of the present invention are also free of other proteins found in the virion.

Chemical synthesis of peptides is described in the following publications: S. B. H. Kent, Biomedical Polymers, eds., E. P. Goldberg and A.Nakajima, (Academic Press, New York), 213-242, (1980); A. R. Mitchell, S. B. H. Kent, M. Engelhard, and R. B. Merrifield, J. Org. Chem., 43, 2845-2852, (1978); J. P. Tam, T.-W. Wong, M. Riemen, F.-S. Tjoeng, and R. B. Merrifield, Tet. Letters, 4033-4036, (1979); S. Mojsov, A. R. Mitchell, and R. B. Merrifield, J. Org. Chem., 45, 555-560, (1980); J. P. Tam, R. D. DiMarchi and R. B. Merrifield, Tet. Letters, 2851-2854, (1981); and S. B. H. Kent, M. Riemen, M. Le Doux and R. B. Merrifield, Proceedings of the IV International Symposium on Methods of Protein Sequence Analysis, (Brookhaven Press, Brookhaven, NY), 1981.

Chemical Synthesis: In the so-called "Merrifield solid phase procedure" the appropriate sequence of L-amino acids is built up from the carboxyl terminal amino acid to the amino terminal amino acid. Starting with the appropriate carboxyl terminal amino acid attached to a polystyrene (or other appropriate) resin via chemical linkage to a chloromethyl group, benzhydrylamine group, or other reactive group of the resin, amino acids are added one by one using the following procedure. The peptide-resin is:

(a) washed with methylene chloride;

(b) neutralized by mixing for 10 minutes at room temperature with 5% (v/v) diisopropylethylamine (or other hindered base) in methylene chloride;

(c) washed with methylene chloride;

(d) an amount of amino acid equal to six times the molar amount of the growing peptide chain is activated by combining it with one-half as many moles of a carbodiimide (e.g., dicyclohexylcarbodiimide, or diisopropylcarbodiimide) for ten minutes at 0°C, to form the symmetric anhydride of the amino acid. The amino acid used should be provided originally as the N-alpha-tert.butyloxycarbonyl derivative, with side chains protected with benzyl esters (e.g. aspartic or glutamic acids), benzyl ethers (e.g.,serine, threonine, cysteine or tyrosine), benzyloxycarbonyl groups (e.g., lysine) or other protecting groups commonly used in peptide synthesis.

(e) the activated amino acid is reacted with the peptide-resin for two hours at room temperature, resulting in addition of the new amino acid to the end of the growing peptide chain.

(f) the peptide-resin is washed with methylene chloride;

(g) the N-alpha-(tert. butyloxycarbonyl) group is removed from the most recently added amino acid by reacting with 30 to 65%, preferably 50% (v/v) trifluoroacetic acid in methylenechloride for 10 to 30 minutes at room temperature;

(h) the peptide-resin is washed with methylene chloride;

(i) steps (a) through (h) are repeated until the required peptide sequence has been constructed.

The peptide is then removed from the resin and simultaneously the side-chain protecting groups are removed, by reaction with anhydrous hydrofluoric acid containing 10% v/v of anisole or other suitable (aromatic) scavenger. Subsequently, the peptide can be purified by gel filtration, ion exchange, high pressure liquid chromatography, or other suitable means.

In some cases, chemical synthesis can be carried out without the solid phase resin, in which case the synthetic reactions are performed entirely in solution. The reactions are similar and well known in the art, and the final product is essentially identical.

Isolation from natural sources: If sufficient quantities of the whole protein antigen are available, a limited portion of the molecule, bearing the desired sequence of amino acids may be excised by any of the following procedures:

(a) Digestion of the protein by proteolytic enzymes, especially those enzymes whose substrate specificity results in cleavage of the protein at sites immediately adjacent to the desired sequence of amino acids;

(b) Cleavage of the protein by chemical means. Particular bonds between amino acids can be cleaved by reaction with specific reagents. Examples include: bonds involving methionine are cleaved by cyanogen bromide; asparaginyl-glycine bonds are cleaved by hydroxylamine;

(c) A combination of proteolytic and chemical cleavages.

It should also be possible to clone a small portion of the DNA, either from natural sources or prepared by synthetic procedures, or by methods involving a combination thereof, that codes for the desired sequence of amino acids, resulting in the production of the peptide by bacteria, or other cells.

Peptides mimicking the antigenic determinant of HBsAg (S region) include the following:

S(135-155) (A. R. Neurath, S. B. H. Kent and N. Strick, "Specificity of Antibodies Elicited by a Synthetic Peptide Having a Sequence in Common With a Fragment of a Virus Protein, the Hepatitis B Surface Antigen", Proc. Natl. Acad. Sci. USA, 79, 7871-7875, Dec. 1982);

(1)

Peptide 1

```
                                X
                                  ╲
                                    Lys
                                    122
                                     |
                                    Thr
                                     |
            Ser  —  Cys  =  Cys  -  Met  -  Thr
             |      137    124               |
            Pro                              Thr
             |                                |
            Tyr                              Ala
             |                                |
            Met  —  Ser  —  Thr  —  Gly  —  Gln
```

Peptide 2 contains 5 additional amino acid residues:

```
            Ser  —  Thr  —  Gly  —  Pro  —  Ser  —  X,
            117                            121
```

G. R. Dreesman, Y. Sanchez, I. Ionescu-Matiu, J. T. Sparrow, H. R. Six, D. L. Peterson, F. B. Hollinger and J. L. Melnick, "Antibody to Hepatitis B Surface Antigen After A Single Inoculation of Uncoupled Synthetic HBsAg Peptides", Nature, 295, 158-160, 1982; and (2) the following peptides:

| POSITION | SEQUENCE |
|---|---|
| 48-81 | Cys-Leu-Gly-Gln-Asn-Ser-Gln-Ser-Pro-Thr-Ser-Asn-His-Ser-Pro-Thr-Ser-Cys-Pro-Pro-Thr-Cys-Pro-Gly-Tyr-Arg-Trp-Met-Cys-Leu-Arg-Arg-Phe-Ile |
| 2-16 | Glu-Asn-Ile-Thr-Ser-Gly-Phe-Leu-Gly-Pro-Leu-Leu-Val-Leu-Gln-Cys |
| 22-35 | Leu-Thr-Arg-Ile-Leu-Thr-Ile-Pro-Gln-Ser-Leu-Asp-Ser-Trp-Cys |
| 38-52 | Ser-Leu-Asn-Phe-Leu-Gly-Gly-Thr-Thr-Val-Cys-Leu-Gly-Gln-Asn |
| 47-52 | Val-Cys-Leu-Gly-Gln-Asn |
| 95-109 | Leu-Val-Leu-Leu-Asp-Tyr-Gln-Gly-Met-Leu-Pro-Val-Cys-Pro-Leu |
| 104-109 | Leu-Pro-Val-Cys-Pro-Leu |

The complexes of the present invention can be utilized to bind synthetic peptide analogues (eliciting protective antibodies) of various viral, bacterial, allergen and parasitic proteins of man and animals, besides synthetic peptide analogues of hepatitis B surface antigen, and especially the novel synthetic peptide analogue of hepatitis B surface antigen containing amino acid sequences corresponding to amino acid sequences in pre-S gene coded region of the HBV.

Accordingly, the complexes of the present invention can be used to bind with synthetic peptide analogues of the following viruses: HTLV III/LAV, influenza hemagglutinin (A/memphis/102/72 strain, A/Eng 1878/69 strain, A/NT/60/68/29c strain, and A/Qu/7/70 strain, Ao/PR8/34, A1/CAM/46, and A2/Singapore/1/57; Type B influenza viruses, e.g. B/Lee 40), fowl plague virus hemagglutinin, vaccinia, polio, rubella, cytomegalovirus, small pox, herpes simplex types I and II, yellow fever, Infectious ectromelia virus, Cowpox virus, Infectious bovine rhinotracheitis virus, Equine rhino- pneumonitis (equine abortion) virus, Malignant catarrh virus of cattle, Feline rhinotracheitis virus, Canine herpes virus, Epstein-Barr virus (associated with infectious mononucleosis and Burkitt lymphoma), Marek's disease virus, Sheep pulmonary adenomatosis (Jaagziekte) virus, Cytomegaloviruses, Adenovirus group, Human papilloma virus, Feline panleucopaenia virus, Mink enteritis virus, African horse sickness virus (9 serotypes), Blue tongue virus (12 serotypes), Infectious pancreatic neurosis virus of trout, Fowl sarcoma virus (various strains), Avian leukosis virus (visceral, erythroblastic and myeloblastic), Osteopetrosis virus, Newcastle disease virus, Parainfluenza virus 1, Parainfluenza virus 2, Parainfluenza virus 3, Parainfluenza 4, Mumps virus, Turkey virus, CANADA/58, Canine distemper virus, Measles virus, Respiratory syncytial virus, e.g., B influenza viruses, e.g., B/Lee/40; Rabies virus; Eastern equinine encephalitis virus; Venezuelan equine encephalitis virus; Western equine encephalitis virus; Yellow fever virus, Dengue type 1 virus (= type 6), Dengue type 2 virus (= type 5); Dengue type 3 virus; Dengue type 4 virus; Japanese encephalitis virus, Kyasanur Forest virus; Louping ill virus; Murray Valley encephalitis virus; Omsk haemorrhagic fever virus (types I and II); St. Louis encephalitis virus; Human rhinoviruses, Foot-and-mouth disease virus; Poliovirus type 1; Enterovirus Polio 2; Enterovirus Polio 3; Avian infectious bronchitis virus; Transmissible gastro-enteritis virus of swine; Lymphocytic choriomeningitis virus; Lassa virus; Machupo virus; Pichinde virus; Tacaribe virus; Papillomavirus; Sindbis virus, and the like.

The complexes of the present invention can also be used to bind synthetic peptide analogues of bacteria, for example, leprosy, tuberculosis, syphilis and gonorrhea.

The complexes of the present invention can also be used to bind synthetic peptide analogues of the following parasites: organisms carrying malaria (P. Falciparum, P. Ovace, etc.), Schistosomiasis, Onchocerca Volvulus and other filiarial parasites, Trypanosomes, Leishmania, Chagas disease, amoebiasis, hookworm, and the like.

The immunogen of the present invention can be utilized as the active component of a vaccine and can be employed with a physiologically acceptable diluent (medium), e.g., phosphate buffered saline. Generally speaking, the synthetic peptide concentration in a physiologically acceptable medium will be between approximately less than 1 miligram and more than 10 micrograms per dose. The use of an adjuvant, however, is not believed to be necessary.

The vaccine can be administered by subcutaneous, intradermal or intramuscular injection. While the preferred route would depend upon the particular vaccine, it is believed that intramuscular injection will be generally suitable. Frequency of administration will vary depending upon the vaccine. Generally speaking, the vaccine will be administered in two doses about one month apart followed by a booster at six months to one year after primary immunization. The subsequent doses or the booster will depend on the level of antibody in the blood as a result of the initial immunization, and in certain instances may be unnecessary.

Vaccines according to the present invention can be used to improve immune response and to overcome non-responsiveness to certain known hepatitis B virus vaccines (e.g., containing no peptides corresponding to amino acid sequences in the pre-S region).

The invention will now be described with reference to the following non-limiting example.

EXAMPLE

Octadecanethiol (8 mg) (Fluka, Hauppauge, New York) and 2-mercaptoethanol (ME; 20 mg) were dissolved in 2 ml of dimethyl sulfoxide (DMSO) at 20°C. After 30 minutes, Spikoside (Isotec AB, Lulea, Sweden 8 mg) was added and the mixture was applied on top of a 40 ml column of "SEPHADEX G-10" (Pharmacia, Piscataway, New Jersey) which had been prewashed with 0.8% sodium acetate, pH 6.7, followed by 2 ml of the same buffer containing 2 mg/ml of Spikoside. After sample application, the column was eluted with the same buffer containing 2 $\mu$g/ml Spikoside. Opalescent fractions corresponding to the void volume of the column were pooled and mixed with 2 mg of the synthetic peptide pre-S (120-145), derived from the pre-S2 sequence of the HBV env proteins subtype adw2 (A. R. Neurath et al, Science, 224, 392-395 (1984), supra; A. R. Neurath and S. B. H. Kent, "Antigenic Structure of Human Hepatitis Viruses in Immunochemistry of Viruses: The Basis for Serodiagnosis and Vaccines", 325-366, Edited by M. H. V. Van Regenmortel & A. R. Neurath. Amsterdam: Elsevier, (1985)) and having a Gly-Gly-Cys spacer at the C-terminal. The peptide had been reduced with ME (100 $\mu$g/ml0 and lyophilized. Sodium ferricyanide (6 mM) was then added in 5 x 40 $\mu$l aliquots within 1 hour at 30°C while maintaining the pH at 6.7. The mixture was then dialyzed against 0.14 M NaCl 0.01 M phosphate pH 7.2 (PBS). The iscoms were separated from excess PBS by centrifugation at 2,000 rpm. They pelleted at 4°C and floated at 20°C. Competition tests with serial dilutions of the synthetic peptide iscom in an enzyme linked immunoassay (ELISA) with pre-S (120-145) linked to $\beta$-galactosidase (A. R. Neurath, S. B. H. Kent & N. Strick, "Specificity of Antibodies Elicited by a Synthetic Peptide Having a Sequence in Common with a Fragment of a Virus Protein, the Hepatitis B Surface Antigen", Proceedings of the National Academy of Sciences, U.S.A., 79, 7871-7875, (1982)), revealed that the attachment of the peptide to the iscoms was complete.

Complexes containing analogs pre-S (21-47) and S (135-155), derived from the pre-S1 and S sequences of the HBV env protein (Neurath et al, 1982, supra; A. R. Neurath, S. B. H. Kent, N. Strick, P. Taylor, & C. E Stevens, "Hepatitis B Virus Contains Pre-S Gene-Encoded Domains", Nature, 315, 154-156, (1985); Neurath and Kent, (1985), supra), respectively, were prepared in the same way, except that in the case of the latter peptide, 0.1 M phosphate pH 8 was used for molecular exclusion chromatography, sodium ferricyanide was not used and the linking of the peptide to iscoms was prolonged to 16 hours. ELISA competition tests revealed that the binding of each of the two peptides to complexes was complete.

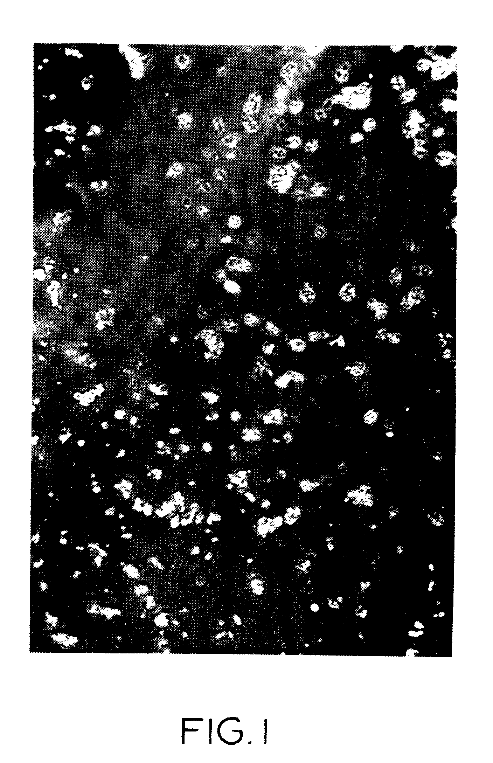

The three distinct complex preparations were redissolved in DMSO and pooled (total volume = 4 ml). Spikoside (4 mg) was added and the mixture was applied to a 40 ml column of "SEPHADEX G-10" prewashed with PBS containing 2 $\mu$g/ ml of Spikoside. Fractions corresponding to the void volume of the column and containing the iscoms with all three distinct peptides attached (Fig. 1) were pooled and used to immunize four rabbits. The iscom dose for each immunization carried out in biweekly intervals corresponded to 125 $\mu$g of each of the three peptides.

Fig 1 is a photomicrograph of iscoms (agglutinated with anti-pre-S (21-47)) containing synthetic peptides corresponding to the pre-S1, pre-S2 and S-regions of the HBV env protein (photographed with a 250x phase contrast objective; bar length = 50 $\mu$). The diameter of the iscoms were approximately 1 to 3 $\mu$.

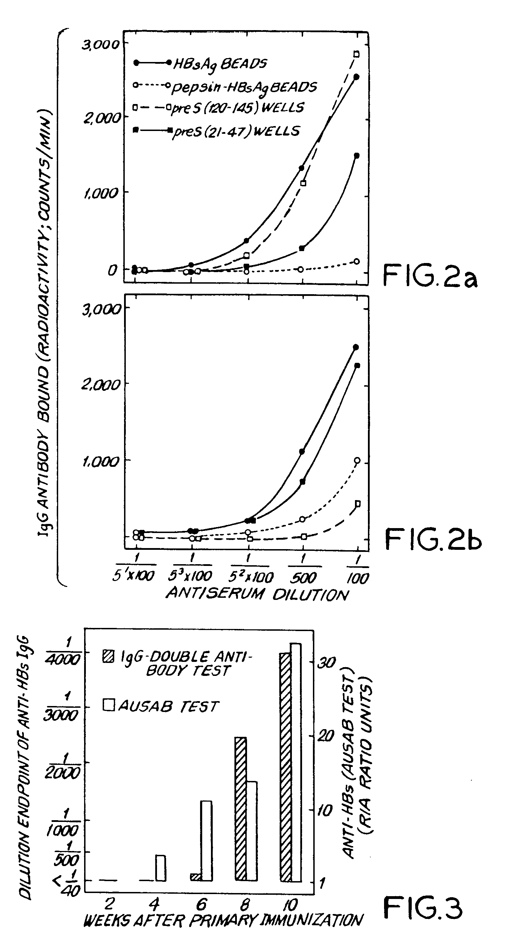

The rabbits responded to all of the synthetic analogs incorporated into the iscoms and consequently reacted with the HBV env proteins present in intact hepatitis B surface antigen (HBsAg; containing pre-S1, pre-S2 and S-protein sequence (Neurath et al, Nature, 315, 154-156, (1985), supra) and in pepsin-treated HBsAG (containing exclusively S-protein, Neurath et al, Nature, 315, 154-156, (1985) , supra). The antisera aggregated HBV particles as observed by electron microscopy (data not shown). Results obtained with the two best responders are shown in Fig. 2.

Fig. 2 represents the results of antibody responses in two rabbits (top, bottom) 10 weeks after primary immunization with iscoms containing synthetic peptides derived from the pre-S1, pre-S2 and S-protein sequences of the HBV env protein, subtype adw2 (Neurath and Kent, (1985), supra). Antibodies were assayed by RIA using $^{125}$I-labeled anti-rabbit IgG as second antibody (Neurath et al, (1982) supra. The antigens used for coating of the solid phase are indicated in the insert. Counts of radioactivity corresponding to the preimmune sera were substracted from counts corresponding to anti-complex serum. Antibody responses to the peptide S (135-155) are not shown in Fig. 2 since they correlate poorly with responses to S-protein (Neurath et al, (1982) , supra; Neurath et al, The Journal of General Virology, 65, 1009-1014, (1984)).

The level of antibodies recognizing HBsAg increased with the number of immunization (Fig. 3), suggesting the development of memory cells.

Fig. 3 shows the kinetics of antibody response to the HBV env proteins in one of the rabbits immunized as described for Fig. 2a. Beads coated with HBsAg containing all envelope proteins as determined by Western blots and by serological assays (Neurath et al, Nature, 315, 154-156, (1985), supra), were used to detect antibodies. The endpoint dilution represent the highest dilution at which the ratio (RIA ratio units) of counts corresponding to the antisera divided by counts corresponding to equally diluted preimmune sera was $\geq 2.1$. Antibodies to S-protein were measured by the AUSAB test (Abbott Laboratories, North Chicago, Illinois) using undiluted sera. Therefore, the scales on the left and right ordinate of Fig. 3 are not comparable.

## Claims

1. A process for prepraring a complex immunogen characterized in that:
(1) in a first step there is linked in a water-miscible organic solvent

(a) a saponin derivative to

(b) an organic compound selected from the group consisting of a $C_{7-24}$-organic compound and a hydrophobic peptide, said organic compound containing one or more active groups selected from the group consisting of -COOH, -CHO, -NH$_2$, and -SH, and said organic compound being soluble in the water-miscible organic solvent;

(2) in a second step the complex formed in (1) is separated by gel filtration from the organic solvent and any excess saponin derivative; and

(3) in a third step one or more synthetic peptides are covalently linked to the linking moiety in the complex separated in (2) through the active moieties, said synthetic peptides corresponding to one or more antigenic determinants.

2. The process according to claim 1, wherein the saponin derivative is Quil A.

3. The process according to claim 1, wherein the water-miscible organic solvent is selected from the group consisting of dimethyl sulfoxide, dimethyl formamide, and ethanol.

4. The process according to claim 1, wherein the organic compound has 12 to 18 carbon atoms.

5. The process according to claim 1, wherein the organic compound has 14 to 18 carbon atoms.

6. The process according to claim 1, wherein the organic compound is a fatty acid selected from the group consisting of stearic acid, oleic acid, palmitic acid, and myristic acid.

7. The process according to claim 1, wherein the organic compound is an aliphatic compound.

8. The process according to claim 7, wherein the aliphatic compound is octadecanethiol.

9. The process according to Claim 1, wherein the synthetic peptides correspond to the segments of the hepatitis B virus envelope protein or analogues thereof.

10. The process according to claim 1, wherein the synthetic peptides are (1) at least one peptide analog from the pre-S1 region of the hepatitis B virus envelope protein, (2) at least one peptide analog from the pre-S2 region of the hepatitis B virus envelope protein, and (3) at least one peptide analog from the S region of the hepatitis B virus envelope protein.

11. The process according to claim 10, wherein synthetic peptide analog (2) is pre-S (120-145).

12. The process according to claim 10, wherein synthetic analog (1) is pre-S (21-47).

13. The process according to claim 10, wherein synthetic analog (3) is S (135-155).

14. A complex immunogen prepared by the process of any one of preceding claims 1 to 13.

15. The use of the complex immunogen according to claim 14 in the preparation of a vaccine protective against viral infection.

16. The use according to claim 15, wherein the viral infection is hepatitis B.

**Patentansprüche**

1. Verfahren zur Herstellung eines Immunogenkomplexes, dadurch gekennzeichnet, daß

(1) zuerst in einem mit Wasser mischbaren organischen Lösungsmittel

a) ein Saponinderivat mit

b) einer organischen Verbindung, ausgewählt aus der Gruppe, die besteht aus einer organischen $C_{7-24}$-Verbindung und einem hydrophoben Peptid, verbunden wird, wobei die organische Verbindung eine oder mehrere aktive Gruppen, ausgewählt aus der aus -COOH, -CHO, -NH$_2$ und -SH bestehenden Gruppe, enthält und wobei die organische Verbindung in dem mit Wasser mischbaren organischen Lösungsmittel löslich ist;

13

EP 0 244 719 B1

(2) in einer zweiten Stufe der in (1) gebildete Komplex durch Gelfiltration von dem organischen Lösungsmittel und überschüssigem Saponinderivat getrennt wird und

(3) in einer dritten Stufe ein oder mehrere synthetische Peptide kovalent an die Bindungsstelle des in (2) abgetrennten Komplexes über aktive Stellen gebunden wird, wobei die synthetischen Peptide einer oder mehreren antigenen Determinanten entsprechen.

2. Verfahren nach Anspruch 1, worin das Saponinderivat Quil A ist.

3. Verfahren nach Anspruch 1, worin das mit Wasser mischbare organische Lösungsmittel ausgewählt ist aus der aus Dimethylsulfoxid, Dimethylformamid und Ethanol bestehenden Gruppe.

4. Verfahren nach Anspruch 1, worin die organische Verbindung 12 bis 18 Kohlenstoffatome aufweist.

5. Verfahren nach Anspruch 1, worin die organische Verbindung 14 bis 18 Kohlenstoffatome aufweist.

6. Verfahren nach Anspruch 1, worin die organische Verbindung ausgewählt ist aus der aus Stearinsäure, Ölsäure, Palmitinsäure und Myristinsäure bestehenden Gruppe.

7. Verfahren nach Anspruch 1, worin die organische Verbindung eine aliphatische Verbindung ist.

8. Verfahren nach Anspruch 7, worin die aliphatische Verbindung Octadecanthiol ist.

9. Verfahren nach Anspruch 1, worin die synthetischen Peptide den Segmenten des Hepatitis B-Virushüllenprotein oder deren Analoga entsprechen.

10. Verfahren nach Anspruch 1, worin die synthetischen Peptide (1) wenigstens ein Peptid analog der Prä-S1-Region des Hepatitis B-Virushüllenproteins, (2) wenigstens ein Peptid analog der Prä-S2-Region des Hepatitis B-Virushüllenproteins und (3) wenigstens ein Peptid analog der S-Region des Hepatitis B-Virushüllenproteins sind.

11. Verfahren nach Anspruch 10, worin das synthetische Peptid, welches (2) entspricht, Prä-S (120-145) ist.

12. Verfahren nach Anspruch 10, worin das synthetische Analogon (1) Prä-S (21-47) ist.

13. Verfahren nach Anspruch 10, worin das synthetische Analogon (3) S (135-155) ist.

14. Immunogenkomplex, hergestellt durch das Verfahren nach einem der Ansprüche 1 bis 13.

15. Verwendung des Immunogenkomplexes nach Anspruch 14 zur Herstellung eines gegen Vireninfektion schützenden Impfstoffs.

16. Verwendung nach Anspruch 15, wobei die Virusinfektion durch das Hepatitis B-Virus erfolgt ist.

**Revendications**

1. Procédé de préparation d'un immunogène complexe, caractérisé en ce que :

(1) dans une première étape, on lie dans un solvant organique miscible à l'eau

(a) un dérivé de saponine à

(b) un composé organique choisi parmi un composé organique en $C_{7-24}$ et un peptide hydrophobe, ledit composé organique contenant un ou plusieurs groupes actifs choisis parmi les groupes -COOH, -CHO, -NH$_2$, et -SH, et ledit composé organique étant soluble dans le solvant organique miscible à l'eau;

(2) dans une seconde étape, le complexe formé à l'étape (1) est séparé du solvant organique et de l'excès de dérivé de saponine par filtration sur gel; et

(3) dans une troisième étape, un ou plusieurs peptides synthétiques sont liés par covalence à la portion liante du complexe séparé à l'étape (2) par l'intermédiaire des groupes actifs, lesdits peptides synthétiques correspondant à un ou plusieurs déterminants antigènes.

14

2. Procédé selon la revendication 1, dans lequel le dérivé de saponine est le Quil A.

3. Procédé selon la revendication 1, dans lequel le solvant organique miscible à l'eau est choisi parmi le diméthylsulfoxide, le diméthylformamide, et l'éthanol.

4. Procédé selon la revendication 1, dans lequel le composé organique possède de 12 à 18 atomes de carbone.

5. Procédé selon la revendication 1, dans lequel le composé organique possède de 14 à 18 atomes de carbone.

6. Procédé selon la revendication 1, dans lequel le composé organique est un acide gras choisi parmi l'acide stéarique, l'acide oléique, l'acide palmitique, et l'acide myristique.

7. Procédé selon la revendication 1, dans lequel le composé organique est un composé aliphatique.

8. Procédé selon la revendication 7, dans lequel le composé aliphatique est l'octadécanethiol.

9. Procédé selon la revendication 1, dans lequel les peptides synthétiques correspondent à des segments de la protéine de l'enveloppe du virus de l'hépatite B ou d'analogues de cette dernière.

10. Procédé selon la revendication 1, dans lequel les peptides synthétiques sont (1) au moins un analogue de peptide de la région pré-S1 de la protéine de l'enveloppe du virus de l'hépatite B, (2) au moins un analogue du peptide de la région pré-S2 de la protéine de l'enveloppe du virus de l'hépatite B, et (3) au moins un analogue de peptide de la région S de la protéine de l'enveloppe du virus de l'hépatite B.

11. Procédé selon la revendication 10, dans lequel l'analogue du peptide synthétique (2) est le peptide pré-S (120-145).

12. Procédé selon la revendication 10, dans lequel l'analogue synthétique (1) est le peptide pré-S (21-47).

13. Procédé selon la revendication 10, dans lequel l'analogue synthétique (3) est le peptide S (135-155).

14. Immunogène complexe préparé par le procédé selon l'une quelconque des revendications précédentes 1 à 13.

15. Utilisation de l'immunogène complexe selon la revendication 14 dans la préparation d'un vaccin protecteur contre une infection virale.

16. Utilisation selon la revendication 15, dans laquelle l'infection virale est l'hépatite B.

FIG. I

FIG.2a

FIG.2b

FIG.3

PRE·S PROTEIN

```
            1          2          3          4          5          6
   1234567890123456789012345678901234567890123456789012345678901 2

1ayw  GHHILGNKIYSMGQNLSTSNPLGFFPDHQLDPAFRANTANPDWDFNPNKDTWPDANKVGAGA
2adyw GHHILGNKSYSMGQNLSTSNPLGFFPDHQLDPAFRANTNNPDWDFNPNKDTWPDANKVGAGA
3adw2 HGGWSSKPRKGMGTNLSVPNPLGFFPDHQLDPAFGANSNNPDWDFNPVKDQWPAANQVGVGA
4adw  LGNKSYSIRKGMGTNLSVPNPLGFLPDHQLDPAFGANSTNPDWDFNPIKDHWPAANQVGVGA
5adr  MGGWSSKPRQGMGTNLSVPNPLGFFPDHQLDPAFGANSNNPDWDFNPNKDQWPEANQVGAGA

            MG  NLS   NPLGF PDHQLDPAF AN   NPDWDFNP KD  WP AN  VG  GA


         6          7          8          9          10         11         12
    34567890123456789012345678901234567890123456789012345678901234

1ayw  FGLGFTPPHGGLLGWSPQAQGILQTLPANPPASTNRQSGRQPTPLSPPLRNTHPQAMQWNS
2adyw FGLGFTPPHGGLLGWSPQAQGIMQTLPANPPASTNRQSGRQPTPLSPPLRTTHPQAMHWNS
3adw2 FGPRLTPPHGGILGWSPQAQGILTTVSTIPPPASTNRQSGRQPTPISPPLRDSHPQAMQWNS
4adw  FGPGLTPPHGGILGWSPQAQGILTTVSTIPPPASTNRQSGRQPTPISPPLRDSHPQAMQWNS
5adr  FGPGFTPPHGGLLGWSPQAQGILTTVPAAPPPASTNRQSGRQPTPISPPLRDSHPQAMQWNS

      FG     TPPHGG  LGWSPQAQGI   T     PPPASTNRQSGRQPTP SPPLR   HPQAM WNS


        12    13         14         15         16         17
    567890123456789012345678901234567890123456789012345678901234

1ayw  TTFHQTLQDPRVRGLYFPAGGSSSGTVNPVLTTASPLSSIFSRIGDPALN
2adyw TTFHQTLQDPRVRGLYFPAGGSSSGTVNPVPTTTSPISSIFSRIGDPALN
3adw2 TAFHQTLQDPRVRGLYLPAGGSSSGTVNPAPNIASHISSISARTGDPVTN
4adw  TALHQALQDPRVRGLYLPAGGSSSGTVNPAPNIASHISSISARTGDPVTI
5adr  TTFHQALLDPRVRGLYFPAGGSSSGTVNPVPTTASPISSIFSRTGDPAPN

      T   HQ L DPRVRGLY PAGGSSSGTVNP       S   SSI    R GDP
```

FIG. 4